# EUROPEAN PATENT SPECIFICATION

(11) **EP 3 147 347 B1**
(45) Date of publication and mention of the grant of the patent: **06.05.2020**
(21) Application number: 16190447.9
(22) Date of filing: 23.09.2016
(51) Int. Cl.: C12M 1/12, B01L 3/00, C12M 1/22

(54) **CULTURE DISH**
KULTURSCHALE
RECIPIENT DE CULTURE

(30) Priority: 25.09.2015 CZ 201531565 U
(43) Date of publication of application: 29.03.2017
(73) Proprietor: Univerzita Palackeho v Olomouci, 77147 Olomouc (CZ)
(72) Inventor: DZUBAK, Petr, 75103 Brodek u Prerova (CZ); HAJDUCH, Marian, 79305 Moravsky Beroun (CZ)
(74) Representative: Hartvichova, Katerina

(56) References cited:
- EP-A2- 0 751 215
- DE-A1- 10 109 290
- US-A1- 2006 281 172
- US-A1- 2008 064 090

## Description

### Field of Art

The present invention relates to a culture dish for preparation of mounts of adherent cell lines on culture glass slides.

### Background Art

Preparation of microscopic mounts of adherent cell lines on culture glass slides is a common technique, used in laboratories dealing with cell and molecular biology. There are obvious drawbacks to this technique, which include burdensome manipulation with the culture glass slides, often leading to their damaging or destroying. Furthermore, it is difficult to ensure during manipulation with the culture dish that the culture glass slides do not shift to a position where they overlap with each other. Once overlapping occurs, the mount is damaged and must be prepared a-new.

Document EP 0751215 shows a culture vessel for use with coverslips, which aims at an easier manipulation of the coverslips when removing the coverslips from the culture vessel. The coverslips are used for culturing cells. The culture vessel contains protuberances projecting upwardly from the surface of the bottom, and said protuberances have a height of 1 to 5 mm. This is considered to prevent adhesion of the coverslips to the cultivation vessel due to surface tension effects of medium and cells. Document US 2006/281172 A1 describes a cell culture vessel for culture of cells showing adherence. The culture apparatus comprises wall elements separating compartments. To improve separation of the cultured cells, the bottom surface is provided with protrusions.

However, the present invention is not concerned with coverslips for merely cultivating cells, it is concerned with preparation of mounts for microscopy. Use of protuberances having the minimum height of 1 mm in culture dishes destined for preparation of microscopy mounts is not optimal because of the high height and would cause blurring and distortion of the image in microscope and therefore hinder control during the process of preparation of the microscopy mounts.

### Disclosure of the Invention

The above-discussed drawbacks of the current solutions are overcome by the present invention. According to the present invention, a culture dish for preparation of adherent cell line mounts on culture glass slides has a bottom which is provided with spacers and distancing elements. The spacers serve for maintaining the culture glass slides in a pre-defined space in which the adherence and cultivation of the cells occurs. The spacers ensure that there is no risk of an accidental shift or overlap of the culture glass slides and damaging of the mounts. The distancing elements prevent adhesion of the thin culture glass slides to the bottom of the culture dish due to capillary forces. A culture glass slide which adheres to the bottom of a culture dish can be removed only with difficulties, often leading to its damaging and breaking. The distancing elements prevent the adhesion and the culture glass slide can easily be removed with the aid of tweezers or of a needle, without any risk of damaging the culture glass slide.

Object of the present invention is thus a culture dish for the preparation of adherent cell line mounts on culture glass slides, wherein the bottom of said culture dish is provided with at least two spacers and with at least six distancing elements.

The spacers are located in distances corresponding to the length of the edge of the culture glass slide, most preferred distances between the spacers are 18 mm and/or 24 mm.

In one embodiment, the height of the spacer is 0.5 mm to 5 mm, preferably the height of the spacer is 1.5 mm.

The height of the distancing elements is in the range of from 0.05 mm to 0.5 mm. Preferably, the height of the distancing elements is 0.1 mm. During the preparation of the mounts, it is necessary to control the quality of the cells and their changes in time. This range of heights for distancing elements allows the use of inverted microscopes for observing the adhered cells within the working range of the common microscope objectives. A larger gap between the bottom and the culture glass slide, such as the gap known from the prior art, filled with the culture medium, leads to transmitted or reflected light reduction, and consequently to blur or distortion of the image. The inventors have found that the range of heights for distancing elements according to the present invention is still sufficient to prevent the adhesion of the culture glass slides and allows smooth manipulation with the culture glass slides, while providing for the desired optical properties of the whole system allowing an unhindered observation of the adhered cells during the process of the preparation of the adherent cell line mounts.

The distancing elements are located in distances which are smaller than the length of the edge of the culture glass slide and greater or equal to 5 mm. The culture glass slides usually have standardized lengths of their edges which are known to a person skilled in the art.

In a preferred embodiment, the culture dish bottom has a shape of a square, a disc, a rectangle or a hexagon, most preferably, the culture dish bottom has a shape of a square having the length of the side corresponding to a multiple of the length of the edge of the culture glass slide. In this most preferred embodiment, the space in the culture dish is utilized most effectively.

In one preferred embodiment, the culture dish further comprises a cover.

The culture dish of the invention may be produced from glass and/or from plastic.

### Brief Description of Drawings

Fig. 1: Top view (A) and cross-sectional views (B, C) of a square-shaped culture dish 1 destined for culture glass slides 4 having the dimensions 24 x 24 mm, showing spacers 2.1 and distancing elements 2.2. (B) is a cross-section view along A-A' and (C) is a detail of cross-sectional view along B-B' with the culture glass slide 4 placed on the distancing elements 2.2 and between the spacers 2.1.
Fig. 2: Orthogonal view of a square-shaped culture dish destined for culture glass slides 4 having the dimensions 24 x 24 mm.
Fig. 3: Orthogonal view of a square-shaped culture dish destined for culture glass slides 4 having the dimensions 24 x 24 mm, with a cover.
Fig. 4: Top view of a hexagonal culture dish 1 destined for culture glass slides 4 having the dimensions 18 x 18 mm, showing spacers 2.1 and distancing elements 2.2.
Fig. 5: Orthogonal view of a hexagonal culture dish destined for culture glass slides 4 having the dimensions 18 x 18 mm.
Fig. 6: Orthogonal view of a hexagonal culture dish destined for culture glass slides 4 having the dimensions 18 x 18 mm, with a cover.
Fig. 7: Top view of a disc-shaped culture dish 1 destined for culture glass slides 4 having the dimensions 18 x 18 mm, showing spacers 2.1 and distancing elements 2.2.
Fig. 8: Orthogonal view of a disc-shaped culture dish 1 destined for culture glass slides 4 having the dimensions 18 x 18 mm.
Fig. 9: Orthogonal view of a disc-shaped culture dish destined for culture glass slides 4 having the dimensions 18 x 18 mm, with a cover.

### Examples

### Example 1: A square-shaped culture dish destined for culture glass slides having the dimensions 24 x 24 mm

An example of a square-shaped culture dish suitable for preparation of mounts on culture glass slides having the dimensions 24 x 24 mm is depicted in the following figures.

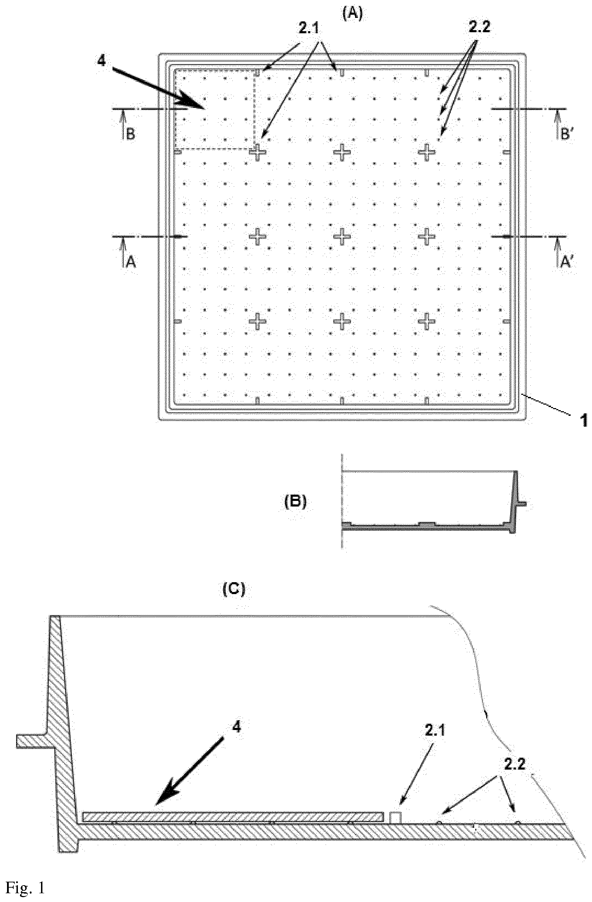

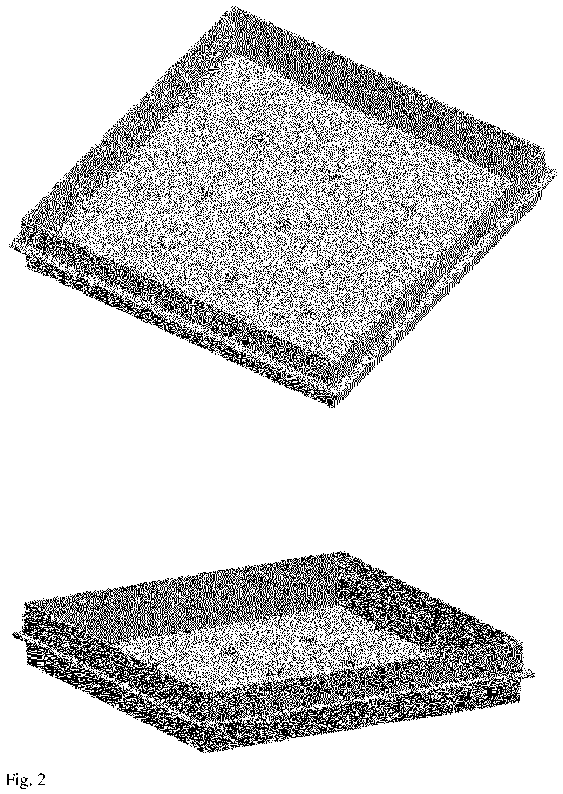

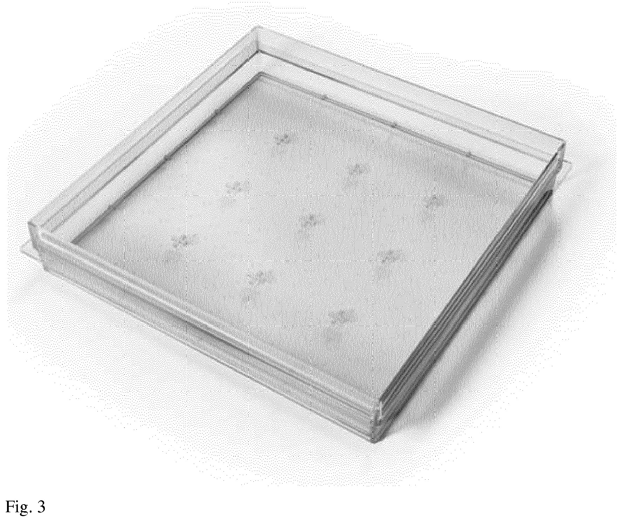

Fig. 1 shows the top view and the cross-sectional views of the square-shaped culture dish 1, which is suitable for preparation of mounts on culture glass slides having the dimensions 24 x 24 mm. Spacers 2.1 having their height 1.5 mm are placed in mutual distances of 24 mm, and distancing elements 2.2 having their height 0.1 mm are placed in mutual distances of 5 mm. Fig. 2 shows orthogonal views on the bottom 2 of the culture dish 1 according to the present invention. Fig. 3 depicts an orthogonal view on the culture dish 1 according to the present invention, including a cover.

### Example 2: A square-shaped culture dish destined for culture glass slides having the dimensions 18 x 18 mm

A square-shaped culture dish suitable for preparation of mounts on culture glass slides having the dimensions 18 x 18 mm is analogical to the culture dish of Example 1. The difference lies in the spacers 2.1, which have their height 0.5 mm, and which are placed in mutual distances of 18 mm.

### Example 3: A hexagonal-shaped culture dish

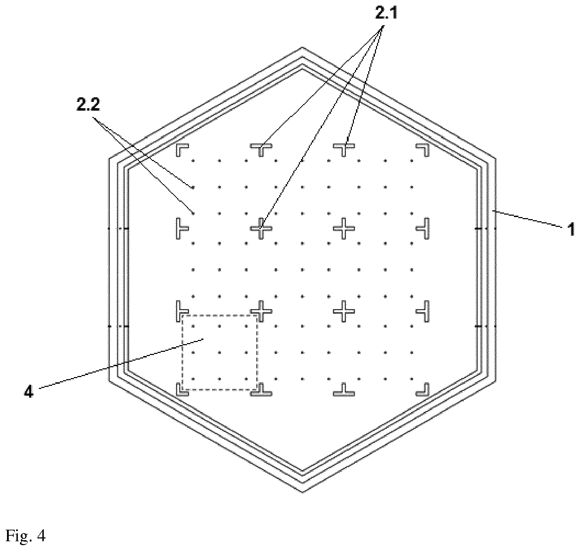

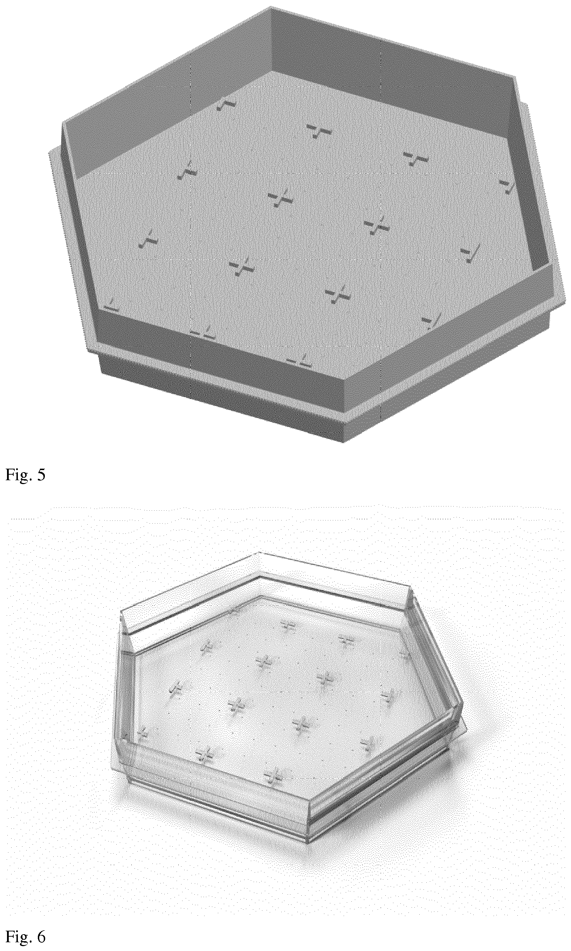

An example of a hexagonal-shaped culture dish suitable for preparation of mounts on culture glass slides having the dimensions 24 x 24 mm is depicted in the following figures. Fig. 4 shows the top view of the hexagonal-shaped culture dish 1, which is suitable for preparation of mounts on culture glass slides 4 having the dimensions 24 x 24 mm. Spacers 2.1 having their height 1.0 mm are placed in mutual distances of 24 mm, and distancing elements 2.2 having their height 0.1 mm are placed in mutual distances of 5 mm. Fig. 5 shows an orthogonal view on the bottom 2 of the culture dish 1 according to the present invention. Fig. 6 depicts an orthogonal view on the culture dish 1 according to the present invention, including a cover 3.

### Example 4: A disc-shaped culture dish

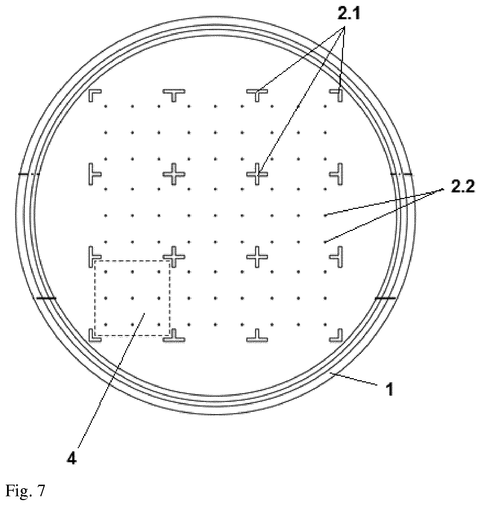

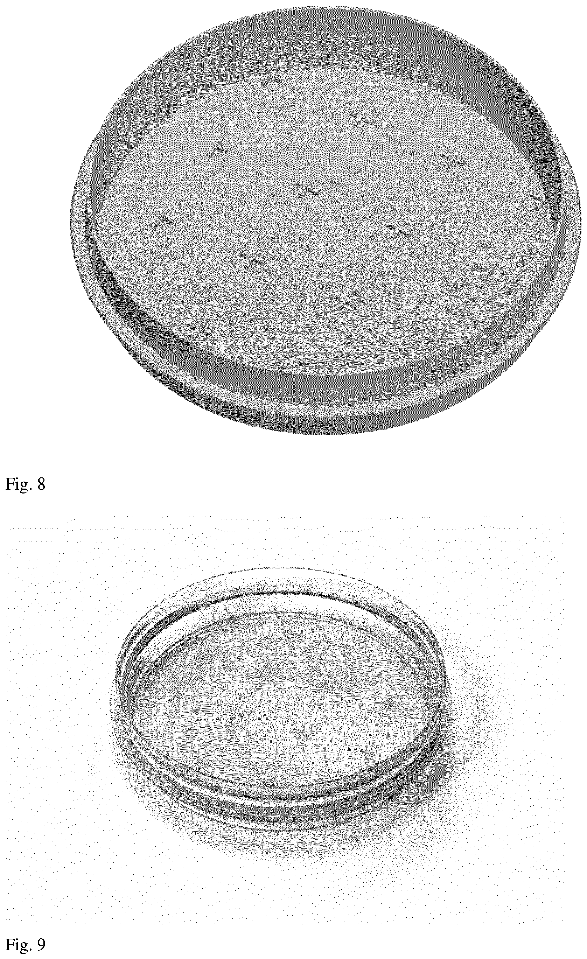

A disc-shaped culture dish suitable for preparation of mounts on culture glass slides having the dimensions 18 x 18 mm is depicted in the following figures. Fig. 7 shows the top view of the disc-shaped culture dish 1, which is suitable for preparation of mounts on culture glass slides 4 having the dimensions 18 x 18 mm. Spacers 2.1 having their height 2.0 mm are placed in mutual distances of 18 mm, and distancing elements 2.2 having their height 0.15 mm are placed in mutual distances of 5 mm. Fig. 8 shows an orthogonal view on the bottom 2 of the culture dish 1 according to the present invention. Fig. 9 depicts an orthogonal view on the culture dish 1 according to the present invention, including a cover 3.

### Example 5: A rectangular culture dish

A rectangular culture dish 1, suitable for preparation of mounts on culture glass slides 4 having the dimensions 18 x 18 mm, was manufactured. Its bottom has a shape of a rectangle having sides 75 mm and 115 mm. Spacers 2.1 having their height 1.0 mm are placed in mutual distances of 18 mm, and distancing elements 2.2 having their height 0.1 mm are placed in mutual distances of 5 mm.

## Claims

1. A culture dish (1) for the preparation of adherent cell line mounts on culture glass slides (4), **characterized in that** the bottom of said culture dish is provided with at least two spacers (2.1) for maintaining the culture glass slides within a pre-defined space, the spacers are located in distances corresponding to the length of the edge of the culture glass slide, and with at least six distancing elements (2.2) for preventing adhesion of the culture glass slides to the culture dish bottom due to capillary forces, said distancing elements having the height in the range of from 0.05 to 0.5 mm, wherein the distancing elements are located in distances which are smaller than the length of the edge of the culture glass slide and greater or equal to 5 mm.

2. The culture dish according to claim 1, wherein the distancing elements are located in mutual distances of 5 mm.

3. The culture dish according to claim 1, wherein the spacers are located in distances of 18 mm and/or 24 mm.

4. The culture dish according to claim 1 or 2, wherein the spacer height is in the range of from 0.5 mm to 5 mm, preferably the spacer height 1.5 mm.

5. The culture dish according to any one of the preceding claims, wherein the distancing element height is 0.1 mm.

6. The culture dish according to any one of the preceding claims, wherein the culture dish bottom has a shape of a square, a disc, a rectangle or a hexagon, preferably the culture dish bottom has a shape of a square having the length of the side corresponding to a multiple of the length of the edge of the culture glass slide.

7. The culture dish according to any one of the preceding claims, wherein the culture dish further comprises a cover.

8. The culture dish according to any one of the preceding claims, wherein the culture dish is made of glass and/or plastic.

## Patentansprüche

1. Kulturschale (1) zur Herstellung anhaftender Zelllinienhalterungen auf Kulturglasobjektträgern (4), **dadurch gekennzeichnet, dass** der Boden der Kulturschale mit mindestens zwei Abstandshaltern (2.1) versehen ist, um die Kulturglasobjektträger zu halten in einem vordefinierten Raum, wobei die Abstandshalter sich in Abständen befinden sich, die der Länge des Randes des Kulturglasobjektträgers entsprechen, und mit mindestens sechs Abstandselementen (2.2) zur Verhinderung des Anhaftens der Kulturglasobjektträger am Kulturschalenboden bedingt Kapillarkräfte, wobei die Abstandselemente eine Höhe im Bereich von 0,05 bis 0,5 mm aufweisen, wobei die Abstandselemente in Abständen angeordnet sind, die kleiner als die Länge des Randes des Kulturglasobjektträgers und größer als oder gleich 5 mm sind.

2. Kulturschale nach Anspruch 1, **dadurch gekennzeichnet, dass** die Abstandselemente in gegenseitigen Abständen von 5 mm angeordnet sind.

3. Kulturschale nach Anspruch 1, **dadurch gekennzeichnet, dass** die Abstandshalter in Abständen von 18 mm und/oder 24 mm angeordnet sind.

4. Kulturschale nach Anspruch 1 oder 2, wobei die Abstandhalterhöhe im Bereich von 0,5 mm bis 5 mm liegt, vorzugsweise ist die Abstandhalterhöhe 1,5 mm.

5. Kulturschale nach einem der vorhergehenden Ansprüche, wobei die Abstandselementhöhe 0,1 mm beträgt.

6. Kulturschale nach einem der vorhergehenden Ansprüche, wobei der Kulturschalenboden die Form eines Quadrats, einer Scheibe, eines Rechtecks oder eines Sechsecks hat, vorzugsweise hat der Kulturschalenboden die Form eines Quadrats mit der Länge von die Seite entsprechend einem Vielfachen der Länge des Randes des Kulturglasobjektträgers.

7. Kulturschale nach einem der vorhergehenden Ansprüche, wobei die Kulturschale ferner einen Deckel aufweist.

8. Kulturschale nach einem der vorhergehenden Ansprüche, wobei die Kulturschale aus Glas und/oder Kunststoff hergestellt ist.

## Revendications

1. Une boîte (1) de culture pour la préparation de montages de lignées cellulaires adhérentes sur des lames (4) de verre de culture, **caractérisé en ce que** le fond de ladit boîte de culture est pourvu d'au moins deux espaceurs (2.1) pour maintenir les lames de verre de culture à l'intérieur d'un espace prédéfini, les espaceurs sont situés à des distances correspondant à la longueur du bord de la lame de verre de culture, et avec au moins six éléments (2.2) de distance pour empêcher l'adhérence des lames de verre de culture au fond de la boîte de culture à cause de forces capillaires, lesdits éléments de distance ayant une hauteur dans la rangée de 0,05 à 0,5 mm, les éléments de distance étant situés à des distances qui sont plus petites que la longueur du bord de la lame de verre de culture et supérieures ou égales à 5 mm.

2. La boîte de culture selon la revendication 1, dans laquel les éléments de distance sont situés à des distances mutuelles de 5 mm.

3. La boîte de culture selon la revendication 1, dans laquel les espaceurs sont situés à des distances de 18 mm et/ou 24 mm.

4. La boîte de culture selon la revendication 1 ou 2, dans laquel la hauteur d'espaceur est dans la rangée de 0,5 mm à 5 mm, de préférence la hauteur d'espaceur est 1,5 mm.

5. La boîte de culture selon l'une quelconque des revendications précédentes, dans laquel la hauteur de l'élément de distance est 0,1 mm.

6. La boîte de culture selon l'une quelconque des revendications précédentes, dans laquel le fond de la boîte de culture a la forme d'un carré, d'un disque, d'un rectangle ou d'un hexagone, de préférence le fond de la boîte de culture a la forme d'un carré ayant la longueur de le côté correspondant à un multiple de la longueur du bord de la lame de verre de culture.

7. La boîte de culture selon l'une quelconque des revendications précédentes, dans laquel la boîte de culture comprend en outre un couvercle.

8. La boîte de culture selon l'une quelconque des revendications précédentes, dans laquel la boîte de culture est en verre et/ou en plastique.
